# EUROPÄISCHE PATENTSCHRIFT

(11) **EP 0 871 604 B1**
(45) Veröffentlichungstag und Bekanntmachung des Hinweises auf die Patenterteilung: **01.12.1999**
(21) Anmeldenummer: 96914194.4
(22) Anmeldetag: 10.05.1996
(51) Int. Cl.: C07C 67/055, C07C 69/15, B01J 23/60, B01J 23/656

(54) **KATALYSATOR UND VERFAHREN ZUR HERSTELLUNG VON VINYLACETAT**
PROCESS AND CATALYST FOR PRODUCING VINYL ACETATE
PROCEDE ET CATALYSEUR POUR PRODUIRE DE L'ACETATE DE VINYLE

(30) Priorität: 23.05.1995 US 448146; 27.06.1995 DE 19523271
(43) Veröffentlichungstag der Anmeldung: 21.10.1998
(73) Patentinhaber: Celanese GmbH, 65926 Frankfurt am Main (DE)
(72) Erfinder: ABEL, Roland, Corpus Christi, TX 78418 (US)
(86) Internationale Anmeldenummer: EP9601994
(87) Internationale Veröffentlichungsnummer: WO9637455

(56) Entgegenhaltungen:
- EP-A- 0 634 214
- US-A- 3 822 308
- US-A- 4 231 897
- US-A- 4 290 921

## Beschreibung

Es ist bekannt, daß man Ethylen in der Gasphase mit Essigsäure und Sauerstoff oder sauerstoffhaltigen Gasen an Palladium/Cadmium/Alkalimetall enthaltenden Festbett-Katalysatoren zu Vinylacetat umsetzen kann. Dabei wird eine Raum-Zeit-Leistung von mehr als 200 g/l•h erhalten (US-A-3 939 199, US-A-4 668 819, US-A-4 902 823, EP-A-0 403 950, US-A-5 225 388, EP-A-0 565 952, EP-A-0 634 208, EP-A-0 634 209, EP-A-0 634 214).

Überraschenderweise wurde nun gefunden, daß derartige Katalysatoren durch Zusatz von mindestens einer Rhenium- und/oder mindestens einer Zirconiumverbindung erheblich verbessert werden, d.h. eine höhere Raum-Zeit-Leistung bei gleicher oder höherer Selektivität der Vinylacetat-Synthese bewirken und langsamer desaktiviert werden.

Ein Gegenstand der Erfindung ist demgemäß ein Verfahren zur Herstellung von Vinylacetat in der Gasphase aus Ethylen, Essigsäure und Sauerstoff oder Sauerstoff enthaltenden Gasen an einem Katalysator, der Palladium und/oder dessen Verbindungen, Cadmiumverbindungen sowie Alkalimetallverbindungen auf einem Träger enthält, dadurch gekennzeichnet, daß der Katalysator zusätzlich mindestens eine Rhenium- und/oder mindestens eine Zirconiumverbindung enthält.

Ein weiterer Gegenstand der Erfindung ist ein Katalysator, der Palladium und/oder dessen Verbindungen, Cadmiumverbindungen sowie Alkalimetallverbindungen auf einem Träger enthält, dadurch gekennzeichnet, daß der Katalysator zusätzlich mindestens eine Rhenium- und/oder mindestens eine Zirconiumverbindung enthält und wobei, wenn der Katalysator Palladium und/oder dessen Verbindungen, Cadmium und mindestens eine Rheniumverbindung enthält, die Alkalimetallverbindung eine Kaliumverbindung sein muß.

Als Träger eignen sich die bekannten inerten Trägermaterialien wie Kieselsäure, Aluminiumoxid, Alumosilikate, Silikate, Titanoxid, Zirkonoxid, Titanate, Siliciumcarbid und Kohle. Besonders geeignet sind Träger dieser Art mit einer spezifischen Oberfläche von 40 bis 350 m²/g (gemessen nach der BET-Methode) und einem mittleren Porenradius von 50 bis 2000 Å (gemessen mit Quecksilber-Porosimetrie), vor allem Kieselsäuren (SiO₂) und SiO₂-Al₂O₃-Gemische. Diese Träger werden in Form von Kugeln, Tabletten, Ringen, Sternen oder anders geformten Teilchen benutzt, deren Durchmesser bzw. deren Länge und Dicke im allgemeinen bei 3 bis 9 mm liegt.

Vorzugsweise liegt das Gesamtporenvolumen des Trägers bei 0,4 - 1,2 ml/g, wobei weniger als 10 % dieses Volumens von "Mikroporen" mit einem Porendurchmesser unter 30 Å (Angström) gebildet werden sollten. Solche Träger können aus aerogenem SiO₂ oder einem aerogenen SiO₂-Al₂O₃-Gemisch hergestellt werden, welches in Form von glasigen Mikrokugeln vorliegt, die beispielsweise durch Flammenhydrolyse von Siliciumtetrachlorid oder eines Siliciumtetrachlorid-Aluminiumtrichlorid-Gemisches in einer Knallgasflamme hergestellt werden können (US-A-3 939 199). Im Handel sind diese Mikrokugeln unter dem Namen ®Aerosil oder ®Cabosil erhältlich.

Besonders bevorzugt ist der Einsatz eines aus solchen Mikrokugeln unter Verwendung von organischen Füllstoffen gepreßten Trägers aus SiO₂ oder SiO₂-Al₂O₃-Gemisch mit einer Oberfläche von 50 - 250 m²/g und einem Porenvolumen von 0,4 - 1,2 ml/g (EP-A-0 403 950). Die Teilchen dieses Trägers haben eine Korngröße von 4 bis 9 mm, wobei 5 bis 20 % des Porenvolumens des Trägers durch Poren mit Radien von 200 bis 3000 Å und 50 bis 90 % des Porenvolumens durch Poren mit Radien von 70 bis 100 Å gebildet wird. Besonders vorteilhaft ist es, wenn diese Trägerteilchen unter Zusatz von einem oder mehreren C₂-C₂₀-Carboxylaten von Li, Mg, Al, Zn, Fe oder Mn als Bindemitteln und unter Zusatz von organischen Füllstoffen (wie Zucker, Harnstoff, höheren Fettsäuren, längerkettigen Paraffinen, mikrokristalliner Cellulose) und Gleitmitteln (wie Kaolin, Graphit, Metallseifen) aus den Mikrokugeln durch Tablettieren oder Extrudieren hergestellt werden (US-A-5 225 388). Anschließend werden die Teilchen in O₂-haltigen Gasen bei etwa 500 - 900°C für etwa 0,25 - 5 Stunden geglüht.

Die katalytisch aktiven Substanzen können in üblicher Weise auf den Träger aufgebracht werden, beispielsweise durch ein- oder mehrmaliges Tränken des Trägers mit einer Lösung der aktiven Substanzen, anschließende Trocknung und gegebenenfalls Reduktion. Jedoch kann man die aktiven Substanzen auch beispielsweise durch ein- oder mehrmaliges Aufsprühen, Aufdampfen oder Tauchen aufbringen oder durch Ausfällung auf dem Träger.

Als Lösungsmittel für die katalytisch aktiven Substanzen sind vor allem Wasser oder unsubstituierte Carbonsäuren mit 2 bis 10 Kohlenstoffatomen, wie Essigsäure, Propionsäure, n- und iso-Buttersäure und die verschiedenen Valeriansäuren geeignet. Wegen ihrer physikalischen Eigenschaften und auch aus wirtschaftlichen Gründen wird als Carbonsäure vorzugsweise Essigsäure eingesetzt. Die zusätzliche Verwendung eines inerten Lösungsmittels ist dann zweckmäßig, wenn eine Carbonsäure eingesetzt wird, in der die Substanzen nicht ausreichend löslich sind. So läßt sich z.B. Palladiumchlorid in einer wäßrigen Essigsäure wesentlich besser lösen als in Eisessig. Als zusätzliche Lösungsmittel kommen diejenigen in Betracht, die inert und mit der Carbonsäure mischbar sind, zum Beispiel Wasser oder Ether, wie Tetrahydrofuran oder Dioxan, aber auch Kohlenwasserstoffe, wie Benzol.

Man kann entweder sogenannte "durchimprägnierte" Katalysatoren herstellen, bei denen die katalytisch wirksamen Metallverbindungen bis zum Kern in die Trägerteilchen eingedrungen sind, oder aber sogenannte "Schalenkatalysatoren", bei denen die Metallsalze nicht bis zum Kern eingedrungen sind, sondern nur in einen mehr oder weniger großen äußeren Teil der Trägerteilchen, d.h. die sogenannte "Schale" der Teilchen. In beiden Fällen können die aufzubringenden Elemente in Form von Lösungen ihrer Verbindungen einzeln aufgebracht werden, oder auch in beliebigen Kombinationen. Vorzugsweise verwendet man Lösungen, die von jedem der aufzubringenden Elemente mindestens eine Verbindung enthalten. Besonders bevorzugt ist der Einsatz einer einzigen Lösung, die von jedem der aufzubringenden Elemente genau eine Verbindung enthält. Wenn im folgenden von "der Lösung" gesprochen wird, so ist damit eine Lösung gemeint, die mindestens eine Verbindung von einem der Elemente Pd, Alkalimetall, Cd, Re, Zr enthält, oder eine Lösung, die von zwei oder mehr dieser Elemente jeweils mindestens eine Verbindung enthält.

Zur Herstellung durchimprägnierter Katalysatoren wird vorzugsweise folgendermaßen vorgegangen (US-A-4 902 823, US-A-3 393 190, US-A-4 668 819):
Die Tränkung des Katalysatorträgers mit der Lösung der aktiven Komponenten wird so vorgenommen, daß das Trägermaterial mit der Lösung überschichtet und gegebenenfalls überschüssige Lösung dann abgegossen oder abfiltriert wird. Mit Rücksicht auf Lösungsverluste ist es vorteilhaft, nur die dem integralen Porenvolumen des Katalysatorträgers entsprechende Menge an Lösung einzusetzen und sorgfältig durchzumischen, damit die Teilchen des Trägermaterials gleichmäßig benetzt werden. Es ist zweckmäßig, den Tränkungsvorgang und das Durchmischen gleichzeitig durchzuführen, beispielsweise in einer Drehtrommel oder einem Taumeltrockner, wobei sich die Trocknung sofort anschließen kann. Weiterhin ist es im allgemeinen zweckmäßig, die Zusammensetzung der zum Tränken des Katalysatorträgers verwendeten Lösung so zu bemessen, daß durch einmaliges Tränken die gewünschte Menge aktiver Stoffe aufgebracht wird. Man kann diese Menge jedoch auch durch mehrere Imprägnierungen aufbringen, wobei vorzugsweise nach jeder Imprägnierung getrocknet wird.

Zur Herstellung von Schalenkatalysatoren wird vorzugsweise nach einer der drei folgenden Methoden vorgegangen, wobei stets eine Lösung von mindestens einer Verbindung von mindestens einem der Elemente Pd, Alkalimetall, Cd, Re und/oder Zr mit einer dynamischen Viskosität von mindestens 0,003 Pa•s, vorzugsweise 0,005 bis 0,009 Pa•s benutzt wird:
1. Die Trägerteilchen werden unter inniger Durchmischung mit der Lösung in Form von Tropfen eines durchschnittlichen Durchmessers von mindestens 0,3 mm oder in Form von Flüssigkeitsstrahlen ein- oder mehrmals bespritzt und nach jedem Bespritzen sofort getrocknet. Die "sofortige" Trocknung bedeutet dabei, daß zügig mit der Trocknung der bespritzten Teilchen begonnen werden muß. Dabei reicht es im allgemeinen, wenn nach dem Ende einer Bespritzung spätestens innerhalb 30 Minuten mit der Trocknung der Teilchen begonnen wird. Das Lösungsvolumen beträgt bei jedem Bespritzen 5 bis 80% des Porenvolumens der Trägerteilchen. Diese Methode wird ausführlich beschrieben in EP-A-0 634 214, auf die hiermit ausdrücklich Bezug genommen wird ("incorporation by reference").
2. Die Trägerteilchen werden unter inniger Durchmischung mit der Lösung ein- oder mehrmals getränkt und nach jedem Tränken sofort getrocknet. Dabei bedeutet die "sofortige" Trocknung dasselbe wie bei der 1. Methode, und das Lösungsvolumen bei jedem Tränken ist 5 bis 80% des Porenvolumens der Trägerteilchen. Diese Methode wird ausführlich beschrieben in EP-A-0 634 209, auf die hiermit ebenfalls ausdrücklich Bezug genommen wird.
3. Die Trägerteilchen werden mit der Lösung ein- oder mehrmals getränkt und nach jedem Tränken getrocknet, aber das Lösungsvolumen ist im Unterschied zur 2.Methode nicht nach oben beschränkt: es beträgt nunmehr bei jedem Tränken mehr als 80% des Porenvolumens. Wegen des größeren Lösungsvolumens ist eine innige Durchmischung nicht unbedingt notwendig, wenngleich im allgemeinen nützlich. Stattdessen muß jetzt die Dauer jeder Tränkung sowie die Zeit bis zum Beginn der darauf folgenden Trocknung, d.h. die Zeit vom Beginn jeder Tränkung bis zum Beginn der darauf folgenden Trocknung, so kurz sein, daß nach Ende der letzten Trocknung eine Schale von 5-80%a des Porenvolumens der Trägerteilchen die katalytisch aktiven Elemente enthält. Wie kurz diese Zeit zu diesem Zweck gewählt werden muß, läßt sich leicht durch Vorversuche ermitteln. Diese Methode wird ausführlich beschrieben in EP-A-0 634 208, auf die hiermit ebenfalls ausdrücklich Bezug genommen wird.

Eine geeignete Methode, um die erzielte Schalendicke bei den hergestellten Schalenkatalysatoren festzustellen, besteht im Aufschneiden einer repräsentativen Zahl von imprägnierten und getrockneten Trägerteilchen und Vermessen der Schalendicken unter dem Mikroskop. Hierbei sollten vorzugsweise weniger als 5% der Teilchen eine Schalendicke haben, die um mehr als 15% vom gewünschten Wert abweicht.

Die Trocknung des getränkten bzw. bespritzten Katalysatorträgers wird sowohl bei durchimprägnierten Katalysatoren als auch bei Schalenkatalysatoren vorzugsweise unter vermindertem Druck (0.1 bis 0.8 bar) durchgeführt. Die Temperatur bei der Trocknung sollte im allgemeinen 50 bis 80°C, vorzugsweise 50 bis 70°C betragen. Weiterhin empfiehlt es sich im allgemeinen, die Trocknung in einem Inertgasstrom, beispielsweise in einem Stickstoff- oder Kohlendioxidstrom vorzunehmen. Der Lösungsmittel-Restgehalt nach der Trocknung sollte vorzugsweise weniger als 8 Gew.-%, insbesondere weniger als 6 Gew.-% betragen.

Die fertigen Katalysatoren sollten folgende Mengen der katalytisch aktiven Elemente enthalten:
Der Palladium-Gehalt beträgt im allgemeinen 0,6 bis 3,5 Gew.-%, vorzugsweise 0,8 bis 3,0 Gew.-%, insbesondere 1,0 bis 2,5 Gew.-%.
Der Alkalielement-Gehalt beträgt im allgemeinen 0,3 bis 10 Gew.-%. Vorzugsweise verwendet man Kalium, und zwar im allgemeinen 0,5 bis 4,0
Gew.-%, vorzugsweise 1,0 bis 3,0 Gew.-%, insbesondere 1,5 bis 2,5 Gew.-%. Der Cadmium-Gehalt beträgt im allgemeinen 0,1 bis 2,5 Gew.-%, vorzugsweise 0,4 bis 2,5 Gew.-%, insbesondere 1,3 bis 2 Gew.-%.

Der Gehalt an Rhenium oder Zirconium beträgt im allgemeinen 0,05 bis 3 Gew.-%, vorzugsweise 0,05 bis 1 Gew.-%, insbesondere 0,05 bis 0,5 Gew.-%. Rhenium und Zirconium können auch gemeinsam im Katalysator vorliegen, wobei dann der Gesamtgehalt beider Elemente innerhalb der genannten Bereiche liegt.

Die angegebenen Prozentzahlen betreffen stets die im Katalysator anwesenden Mengen der Elemente Pd, Alkalielement, Cd, Zr und/oder Re, bezogen auf die Gesamtmasse des Katalysators (aktive Elemente plus Anionen plus Trägermaterial).

Geeignet zum Aufbringen auf den Träger sind alle Verbindungen von Palladium, Cadmium, einem Alkalimetall, Rhenium und Zirconium, die löslich sind und keine für den Katalysator giftigen Bestandteile, wie z.B. Schwefel enthalten; bevorzugt sind die Acetate und die Chloride. Dabei muß aber im Fall der Chloride sichergestellt werden, daß die Chloridionen vor dem Einsatz des Katalysators zur Vinylacetat-Synthese entfernt werden. Dies geschieht durch Auswaschen des dotierten Trägers, z.B. mit Wasser, nachdem beispielsweise das als Chlorid aufgebrachte Palladium in eine unlösliche Form überführt wurde, etwa durch Reduktion und/oder durch Fällung mit Hydroxiden.

Als Verbindungen des Palladiums besonders geeignet sind die Carboxylate, vorzugsweise die Salze der aliphatischen Monocarbonsäuren mit 2 bis 5 Kohlenstoffatomen, etwa das Acetat, das Propionat oder das Butyrat. Weiter sind beispielsweise geeignet das Nitrat, Nitrit, Oxidhydrat, Oxalat, Acetylacetonat, Acetoacetat. Wegen seiner guten Löslichkeit und Zugänglichkeit ist Palladiumacetat die besonders bevorzugte Palladiumverbindung.

Als Alkalimetallverbindung wird vorzugsweise mindestens eine K-, Rb- oder Cs- Verbindung eingesetzt, insbesondere mindestens eine K-Verbindung. Geeignet als Verbindungen sind vor allem Carboxylate, insbesondere Acetate und Propionate. Geeignet sind auch Verbindungen, die unter den Reaktionsbedingungen in das Acetat übergehen, wie etwa das Hydroxid, das Oxid oder das Carbonat.
Als Cadmiumverbindung ist vor allem das Acetat geeignet.
Als Zirconiumverbindungen sind vor allem geeignet das Acetat und das
Acetylacetonat.
Als Rheniumverbindungen sind Re₂O₇ und (NH₄)ReO₄ besonders geeignet.

Falls eine Reduktion der Palladiumverbindung durchgeführt wird, was manchmal nützlich ist, so kann dafür ein gasförmiges Reduktionsmittel eingesetzt werden. Dafür kommen beispielsweise Wasserstoff, Methanol, Formaldehyd, Ethylen, Propylen, Isobutylen, Butylen und andere Olefine in Frage. Die Reduktionstemperatur liegt im allgemeinen zwischen 40 und 260°C, vorzugsweise zwischen 70 und 200°C. Im allgemeinen ist es zweckmäßig, ein mit Inertgas verdünntes Reduktionsmittel zu verwenden, das 0,01 bis 50 Vol.-%, vorzugsweise 0,5 bis 20 Vol.-% Reduktionsmittel enthält. Als Inertgas können beispielsweise Stickstoff, Kohlendioxid oder ein Edelgas verwendet werden. Die Menge des Reduktionsmittels richtet sich nach der Palladiummenge; das Reduktionsäquivalent soll mindestens das 1- bis 1,5-fache des Oxydationsäquivalents betragen, jedoch schaden größere Mengen Reduktionsmittel nicht. Die Reduktion wird im Anschluß an die Trocknung vorgenommen.

Die Herstellung des Vinylacetats erfolgt durch Leiten von Essigsäure, Ethylen und Sauerstoff oder Sauerstoff enthaltenden Gasen bei Temperaturen von 100 bis 220°C, vorzugsweise 120 bis 200°C, und bei Drucken von 1 bis 25 bar, vorzugsweise 1 bis 20 bar, über den fertigen Katalysator, wobei nicht umgesetzte Komponenten im Kreis geführt werden können. Zweckmäßig hält man die Sauerstoffkonzentration unter 10 Vol.-% (bezogen auf das essigsäurefreie Gasgemisch). Manchmal ist jedoch auch eine Verdünnung mit inerten Gasen wie Stickstoff oder Kohlendioxid vorteilhaft. Besonders Kohlendioxid eignet sich zur Verdünnung bei einer Kreislauf-Fahrweise, da es in geringen Mengen während der Reaktion gebildet wird.

Mit Hilfe der erfindungsgemäßen Katalysatoren wird eine höhere Raum-Zeit-Leistung und eine gleiche oder höhere Selektivität bei längerer Standzeit des Katalysators erzielt als mit Katalysatoren, die kein Rhenium oder Zirconium enthalten.

Die folgenden Beispiele sollen die Erfindung erläutern. Die Prozentangaben der Elemente Pd, Cd, Zr, Re und K sind Gewichtsprozente, bezogen auf die Gesamtmasse des Katalysators.

Als Katalysatorträger wurde SiO₂ in Form von Tabletten mit einem Durchmesser und einer Höhe von jeweils 6 mm verwendet. Die Tabletten waren aus ®Aerosil-Pulver mit Hilfe von Magnesiumstearat als Bindemittel gemäß US-A-5 225 388 gepreßt worden. Die Oberfläche des Trägers betrug 120 mg²/g, sein Porenvolumen 0,784 ml/g und sein Schüttgewicht 500 g/l. Das Porenvolumen von 1 l Träger war 392 ml.

### I. Durchimprägnierte Katalysatoren

### Vergleichsbeispiel 1

1 l Kieselsäureträger wurden mit einer Lösung von 24,3 g Palladiumacetat, 21,3 g Cadmiumacetat und 23,8 g Kaliumacetat in 392 ml Eisessig (Lösungsvolumen = 100% des Porenvolumens des Trägers) bei 60°C getränkt. Anschließend wurde in einem Trockenschrank bei 200 mbar unter Stickstoff bis zu einem Restgehalt an Essigsäure von 6-Gew.-% getrocknet; die Trocknungstemperatur betrug 65°C. Der fertige Katalysator enthielt 2,3 Gew.-% Pd, 1,8 Gew.-% Cd und 1,9 Gew.-% K. Er war vollständig, d.h. bis in den Kern, durchimprägniert.

Es wurden 50 ml dieses Katalysators in ein Reaktionsrohr von 8 mm Innendurchmesser und einer Länge von 1,5 m eingefüllt. Dann wurde bei einem Druck von 8 bar (Reaktoreingang) und einer Katalysatortemperatur von 150°C das umzusetzende Gas mehrere Tage über den Katalysator geleitet. Dieses Gas bestand aus 27 Vol.-% Ethylen, 55 Vol.-% Stickstoff, 12 Vol.-% Essigsäure und 6 Vol.-% Sauerstoff. Die Ergebnisse sind aus der Tabelle 1 ersichtlich. In dieser Tabelle bedeutet "relative Geschwindigkeit des Leistungsabfalls" den Quotienten aus Leistungsabfall (= Anfangsleistung des Versuchs minus Endleistung des Versuchs) und Versuchsdauer, relativ zum Quotienten des in Vergleichsbeispiel 1 benutzten Katalysators. Dieser Katalysator hat also den Quotienten (= relative Geschwindigkeit des Leistungsabfalls) 1.

### Beispiel 1a

Es wurde verfahren wie in Vergleichsbeispiel 1, mit der Ausnahme, daß die Lösung zusätzlich 7,5 g Zirconiumacetylacetonat enthielt und die Eisessig-Menge 389 ml betrug. Die Ergebnisse sind aus der Tabelle 1 ersichtlich.

### Beispiel 1b

1 l des wie in Vergleichsbeispiel 1 hergestellten Katalysators wurde mit einer Lösung von 4,2 g Re₂O₇ in 308 ml Wasser (Lösungsvolumen = 100% des Porenvolumens des Katalysators) bei Raumtemperatur getränkt. Anschließend wurde wie in Vergleichsbeispiel 1 getrocknet, bis ein Wasserrestgehalt von 6 Gew.-% erreicht war. Die Austestung erfolgte wie in Vergleichsbeispiel 1. Die Ergebnisse sind aus der Tabelle 1 ersichtlich.

**Tabelle 1**

| (Durchimprägnierte Katalysatoren) | | | |
|---|---|---|---|
| | Leistung * (g/lh) | Selektivität (%) | relative Geschwindigkeit des Leistungsabfalls |
| Vergleichsbeispiel 1 | 813 | 94,3 | 1 |
| Beispiel 1a | 870 | 94,5 | 0,7 |
| Beispiel 1b | 840 | 94,8 | 0,7 |

| | | | |
|---|---|---|---|
| * Anfangsleistung (Gramm Vinylacetat pro Liter Katalysator und Stunde) | | | |

### II. Schalenkatalysatoren

### Vergleichsbeispiel 2

Bei 65°C wurden 25,3 g Palladiumacetat, 25 g Cadmiumacetat und 25,3 g Kaliumacetat in 130,0 ml wasserfreier Essigsäure (Eisessig) gelöst (Lösungsvolumen = 33% des Porenvolumens) und die hochviskose Lösung (7mPa•s) in eine auf 65°C vorgewärmte Vorlage eingefüllt. 1 l Katalysatorträger wurden ebenfalls auf 65°C erwärmt und in einen Kolben gegeben. Dann wurden die Trägerteilchen mit der gesamten Imprägnierlösung übergossen und solange innig durchmischt, bis diese Lösung vollständig von den Teilchen aufgesaugt worden war. Dieser Vorgang war nach 3 Minuten beendet.

Anschließend wurde getrocknet wie im Vergleichsbeispiel 1. Der fertige Katalysator enthielt 2,3 Gew.-% Pd, 1,8 % Gew.-Cd und 1,9 Gew.-% K. Die Schalendicke betrug 0,8 mm.

Die Austestung erfolgte wie in Vergleichsbeispiel 1. Die Ergebnisse sind aus der Tabelle 2 ersichtlich. Die "relative Geschwindigkeit des Leistungsabfalls" ist definiert wie in Vergleichsbeispiel 1 angegeben, d.h. wieder relativ zu dem dort benutzten Katalysator.

### Beispiel 2a

Es wurde verfahren wie in Vergleichsbeispiel 2, mit der Ausnahme, daß die Lösung zusätzlich 7,0 g Zirconiumacetylacetonat enthielt. Die Schalendicke betrug 0,8 mm. Die Ergebnisse sind aus der Tabelle 2 ersichtlich.

### Beispiel 2b

1 l des wie in Vergleichsbeispiel 2 hergestellten Katalysators wurden mit einer Lösung von 3,5 g Re₂O₇ in 300 ml Wasser (Lösungsvolumen = 100% des Porenvolumens des Katalysators) bei Raumtemperatur getränkt. Anschließend wurde wie in Vergleichsbeispiel 1 getrocknet, bis ein Wasserrestgehalt von 6 Gew.-% erreicht war. Die Austestung erfolgte wie in Vergleichsbeispiel 1. Die Ergebnisse sind aus der Tabelle 2 ersichtlich.

**Tabelle 2**

| (Schalenkatalysatoren) | | | |
|---|---|---|---|
| | Leistung * (g/lh) | Selektivität (%) | relative Geschwindigkeit des Leistungsabfalls |
| Vergleichsbeispiel 2 | 922 | 95,8 | 1,4 |
| Beispiel 2a | 950 | 96,1 | 0,9 |
| Beispiel 2b | 940 | 96,0 | 1,0 |

| | | | |
|---|---|---|---|
| * Anfangsleistung (Gramm Vinylacetat pro Liter Katalysator und Stunde) | | | |

## Patentansprüche

1. Verfahren zur Herstellung von Vinylacetat in der Gasphase aus Ethylen, Essigsäure und Sauerstoff oder Sauerstoff enthaltenden Gasen an einem Katalysator, der Palladium und/oder dessen Verbindungen, Cadmium sowie Alkalimetallverbindungen auf einem Träger enthält, dadurch gekennzeichnet, daß der Katalysator zusätzlich mindestens eine Rhenium-und/oder mindestens eine Zirconiumverbindung enthält.

2. Verfahren nach Anspruch 1, dadurch gekennzeichnet, daß der Katalysator mindestens eine Kaliumverbindung enthält.

3. Verfahren nach Anspruch 1 oder 2, dadurch gekennzeichnet, daß der Katalysator 0,05 Gew.-% bis 3 Gew.-% Rhenium und/oder Zirconium, bezogen auf die Gesamtmasse des Katalysators, enthält.

4. Verfahren nach einem der Ansprüche 1 bis 3, dadurch gekennzeichnet, daß der Katalysator 0,05 Gew.-% bis 1 Gew.-% Rhenium und/oder Zirconium, bezogen auf die Gesamtmasse des Katalysators, enthält.

5. Verfahren nach einem der Ansprüche 1 bis 4, dadurch gekennzeichnet, daß der Katalysator 0,05 Gew.-% bis 0,5 Gew.-% Rhenium und/oder Zirconium, bezogen auf die Gesamtmasse des Katalysators, enthält.

6. Katalysator, der Palladium und/oder dessen Verbindungen, Cadmium sowie Alkalimetallverbindungen auf einem Träger enthält, dadurch gekennzeichnet, daß der Katalysator zusätzlich mindestens eine Rhenium- und/oder mindestens eine Zirconiumverbindung enthält und wobei, wenn der Katalysator Palladium und/oder dessen Verbindungen, Cadmium und mindestens eine Rheniumverbindung enthält, die Alkalimetallverbindung eine Kaliumverbindung sein muß.

7. Katalysator nach Anspruch 6, dadurch gekennzeichnet, daß der Katalysator mindestens eine Kaliumverbindung enthält.

8. Katalysator nach Anspruch 6 oder 7, dadurch gekennzeichnet, daß der Katalysator 0,05 Gew.-% bis 3 Gew.-% Rhenium und/oder Zirconium, bezogen auf die Gesamtmasse des Katalysators, enthält.

9. Katalysator nach einem der Ansprüche 6 bis 8, dadurch gekennzeichnet, daß der Katalysator 0,05 Gew.-% bis 1 Gew.-% Rhenium und/oder Zirconium, bezogen auf die Gesamtmasse des Katalysators, enthält.

10. Katalysator nach einem der Ansprüche 6 bis 9, dadurch gekennzeichnet, daß der Katalysator 0,05 Gew.-% bis 0,5 Gew.-% Rhenium und/oder Zirconium, bezogen auf die Gesamtmasse des Katalysators, enthält.

## Claims

1. A process for preparing vinyl acetate in the gas phase from ethylene, acetic acid and oxygen or oxygen-containing gases over a catalyst comprising palladium and/or its compounds, cadmium and alkali metal compounds on a support, wherein the catalyst additionally contains at least one rhenium and/or at least one zirconium compound.

2. The process as claimed in claim 1, wherein the catalyst contains at least one potassium compound.

3. The process as claimed in claim 1 or 2, wherein the catalyst contains from 0.05% by weight to 3% by weight of rhenium and/or zirconium, based on the total mass of the catalyst.

4. The process as claimed in any one of claims 1 to 3, wherein the catalyst contains from 0.05% by weight to 1% by weight of rhenium and/or zirconium, based on the total mass of the catalyst.

5. The process as claimed in any one of claims 1 to 4, wherein the catalyst contains from 0.05% by weight to 0.5% by weight of rhenium and/or zirconium, based on the total mass of the catalyst.

6. A catalyst comprising palladium and/or its compounds, cadmium and alkali metal compounds on a support, wherein the catalyst additionally contains at least one rhenium and/or at least one zirconium compound, and, if the catalyst comprises palladium and/or its compounds, cadmium and at least one rhenium compound, the alkali metal compound has to be a potassium compound.

7. A catalyst as claimed in claim 6, wherein the catalyst contains at least one potassium compound.

8. A catalyst as claimed in claim 6 or 7, wherein the catalyst contains from 0.05% by weight to 3% by weight of rhenium and/or zirconium, based on the total mass of the catalyst.

9. A catalyst as claimed in any one of claims 6 to 8, wherein the catalyst contains from 0.05% by weight to 1% by weight of rhenium and/or zirconium, based on the total mass of the catalyst.

10. A catalyst as claimed in any one of claims 6 to 9, wherein the catalyst contains from 0.05% by weight to 0.5% by weight of rhenium and/or zirconium, based on the total mass of the catalyst.

## Revendications

1. Procédé pour la préparation d'acétate de vinyle en phase gazeuse à partir d'éthylène, d'acide acétique et d'oxygène ou de gaz contenant de l'oxygène sur un catalyseur qui contient du palladium et/ou des composés de celui-ci, du cadmium ainsi que des composés de métaux alcalins sur un support, caractérisé en ce que le catalyseur contient en plus au moins un composé du rhénium et/ou au moins un composé du zirconium.

2. Procédé selon la revendication 1, caractérisé en ce que le catalyseur contient au moins un composé du potassium.

3. Procédé selon la revendication 1 ou 2, caractérisé en ce que le catalyseur contient de 0,05 % en poids à 3 % en poids de rhénium et/ou de zirconium, rapportés à la masse totale du catalyseur.

4. Procédé selon l'une quelconque des revendications 1 à 3, caractérisé en ce que le catalyseur contient de 0,05 % en poids à 1% en poids de rhénium et/ou de zirconium, rapportés à la masse totale du catalyseur.

5. Procédé selon l'une quelconque des revendications 1 à 4, caractérisé en ce que le catalyseur contient de 0,05 % en poids à 0.5 % en poids de rhénium et/ou de zirconium, rapportés à la masse totale du catalyseur.

6. Catalyseur qui contient du palladium et/ou des composés de celui-ci, du cadmium ainsi que des composés de métaux alcalins sur un support. caractérisé en ce que le catalyseur contient en plus au moins un composé du rhénium et/ou au moins un composé du zirconium, et le composé de métal alcalin devant être un composé du potassium lorsque le catalyseur contient du palladium et/ou des composés de celui-ci, du cadmium et au moins un composé du rhénium.

7. Catalyseur selon la revendication 6, caractérisé en ce que le catalyseur contient au moins un composé du potassium.

8. Catalyseur selon la revendication 6 ou 7, caractérisé en ce que le catalyseur contient de 0,05 % en poids à 3 % en poids de rhénium et/ou de zirconium, rapportés à la masse totale du catalyseur.

9. Catalyseur selon l'une quelconque des revendications 6 à 8, caractérisé en ce que le catalyseur contient de 0,05 % en poids à 1 % en poids de rhénium et/ou de zirconium. rapportés à la masse totale du catalyseur.

10. Catalyseur selon l'une quelconque des revendications 6 à 9, caractérisé en ce que le catalyseur contient de 0,05 % en poids à 0,5 % en poids de rhénium et/ou de zirconium, rapportés à la masse totale du catalyseur.
